# FASCICULE DE BREVET EUROPEEN

(11) **EP 1 372 582 B1**
(45) Date de publication et mention de la délivrance du brevet: **03.12.2008**
(21) Numéro de dépôt: 02724384.9
(22) Date de dépôt: 27.03.2002
(51) Int. Cl.: A61K 8/49, A61Q 5/10

(54) **COMPOSITION POUR LA TEINTURE D'OXYDATION CONTENANT 3-AMINO PYRAZOLO-1¬1,5-A -PYRIDINE ET 2-AMINO-3HYDROXYPYRIDINE**
OXIDATIONSFÄRBEMITTEL ENTHALTEND 3-AMINOPYRAZOLO(1,5-A)PYRIDIN UND 2-AMINO-3-HYDROXYPYRIDIN
OXIDATION DYEING COMPOSITION CONTAINING 3-AMINO PYRAZOLO- 1,5-A PYRIDINE AND 2-AMINO-3-HYDROXY PYRIDINE

(30) Priorité: 27.03.2001 FR 0104092
(43) Date de publication de la demande: 02.01.2004
(73) Titulaire: L'ORÉAL, 75008 Paris (FR)
(72) Inventeur: KRAVTCHENKO, Sylvain, F-92600 Asnières (FR); LAGRANGE, Alain, F-77700 Coupvray (FR)
(74) Mandataire: Wattremez, Catherine
(86) Numéro de dépôt international: PCT/FR2002/001060
(87) Numéro de publication internationale: WO 2002/076416

(56) Documents cités:
- EP-A- 0 926 149
- WO-A-01/35917
- DE-A- 2 714 831
- GB-A- 1 026 978
- GB-A- 1 544 587
- US-A- 5 457 200

## Description

L'invention a pour objet de nouvelles compositions pour la teinture d'oxydation des fibres kératiniques comprenant au moins une 3-amino pyrazolo-[1,5-a]-pyridine à titre de base d'oxydation et au moins un coupleur du type 2-amino-3-hydroxy pyridine, un procédé de teinture ainsi qu'un dispositif de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, appelés bases d'oxydation, sont des composés incolores ou faiblement colorés qui, associés à des produits oxydants, peuvent donner naissance par un processus de condensation oxydative à des composés colorés et colorants. Ces composés ont pour point commun de posséder un groupement amino et un groupement hydroxyle ou deux groupements amino, ce qui leur confère leur caractère de base d'oxydation.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possibles, c'est à dire permettre d'obtenir des écarts de coloration les plus faibles possibles tout au long d'une même fibre kératinique, qui peut être en effet différemment sensibilisée (i.e. abîmée) entre sa pointe et sa racine.

Les pyridines sont déjà connues pour la teinture d'oxydation des fibres kératiniques comme bases d'oxydation. Par exemple, les brevets GB 1 026 978 et GB 1 153 196 proposent d'utiliser la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamine pyridine comme base d'oxydation.

De plus, il est connu d'utiliser certains coupleurs du type pyridine pour la teinture d'oxydation des fibres kératiniques par exemples dans les demandes de brevets DE 3132885, DE 3233540, et DE 1149496.

L'invention a pour but de développer de nouvelles compositions tinctoriales permettant d'obtenir une nouvelle gamme de couleurs et ne présentant pas les inconvénients des teintures de la technique antérieure. En particulier, invention a pour but de développer des compositions qui conduisent à des colorations très puissantes, particulièrement résistantes aux diverses agressions que peuvent subir les cheveux (lumière, intempéries, lavage, ondulation permanente, transpiration, frottements), peu sélectives et présentant une bonne innocuité.

A cet effet, l'invention a pour objet une composition pour la teinture d'oxydation des fibres kératiniques, comprenant dans un milieu approprié pour la teinture,
- à titre de base d'oxydation au moins une 3-amino pyrazolo-[1,5-a]-pyridine de formule (I) suivante et/ou un de ses sels d'addition avec un acide ou avec une base: 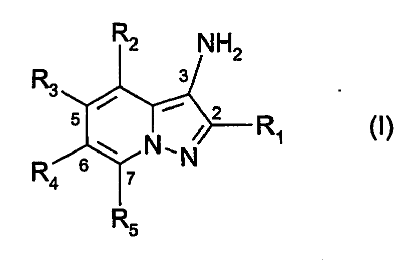 dans laquelle :
   - R₁, R₂, R₃, R₄ et R₅, identiques ou différents, représentent un atome d'hydrogène ou d'halogène ; un radical -NHSO₃H ; un radical hydroxyle ; un radical alkyle ; un radical alkoxy ; un radical alkylthio ; un radical amino ; un radical monoalkylamino ; un radical dialkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle ; un radical nitro ; un radical aryle ; un radical acyle en C1-C4 ; un radical alkoxy(C₁-C₄)carbonyle ; un radical carboxamido ; un radical cyano ; un radical - CO₂H, un radical -SO₃H ; un radical -PO₃H₂ ; un radical -PO₄H₂; ou un groupement de formule (II) suivante: 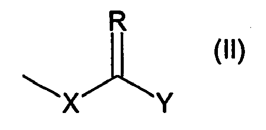 dans laquelle X et R représentent indépendamment l'un de l'autre un atome d'oxygène, un groupement NH ou N(C₁-C₄)alkyle, et Y représente un radical hydroxyle, amino, alkyle, alkoxy, alkylamino, ou dialkylamino, et
- au moins un coupleur pyridinique de formule (III) suivante : 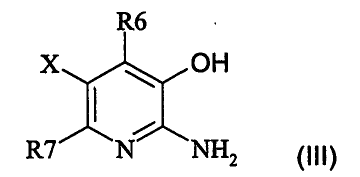 dans laquelle
   - R6 et R7, identiques ou différents, représentent un atome d'hydrogène ; un radical alkyle en C1-C4, et
   - X est un atome d'hydrogène ou d'halogène.

Dans les composés de formule (I) ou (III) utiles pour la présente invention, le terme alkyle utilisé pour les radicaux alkyle ainsi que pour les groupements comportant une partie alkyle signifie, sauf indication différente, une chaîne carbonée, linéaire ou ramifiée, comportant de 1 à 30 atomes de carbone pouvant être interrompue par un ou plusieurs atomes d'oxygène, de soufre ou d'azote, et pouvant être substituée par un ou plusieurs groupes choisis parmi les atomes d'halogène tel le chlore, le brome, l'iode et le fluor ; les hétérocycles ; les radicaux aryle, hydroxyle, alcoxyle, amino, acyle, carboxamido, -CO₂H,-SO₃H, -PO₃H₂, -PO₄H₂, -NHSO₃H, sulfonamide, monoalkylamino, trialkylammonium, ou bien encore par un radical dialkylamino dans lequel les deux groupements alkyle peuvent former, conjointement avec l'atome d'azote dudit groupement dialkyl(C₁-C₄)amino auquel ils sont liés, un cycle pouvant être interrompu par un ou plusieurs atomes d'azote, d'oxygène ou de soufre.

De même, selon l'invention, dans la formule (I), le terme alcoxy utilisé pour les radicaux alcoxy ainsi que pour les groupements comportant une partie alcoxy, signifie sauf indication différente, une chaîne O-alkyle, le terme alkyle ayant la signification indiquée ci dessus.

Selon l'inventon, pour la formule (I), one entend par hétérocycle, un cycle aromatique ou non contenant 5, 6 ou 7 sommets, et de 1 à 3 hétéroatomes choisis parmi les atomes d'azote, de soufre et d'oxygène. Ces hétérocycles peuvent être condensés sur d'autres hétérocycles ou sur un groupement phényle. Ils peuvent être substitués par un atome d'halogène ; un radical alkyle ; un radical alcoxy; un radical hydroxyle ; un radical amino ; un radical alkylamino ; dialkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre. Ces hétérocycles peuvent, en outre, être quatemisés par un radical alkyle. Les termes alkyle et alcoxy ont les significations indiqués ci-dessus.

Parmi ces hétérocycles, on peut notamment citer à titre d'exemple les cycles : thiadiazole, triazole, isoxazole, oxazole, azaphosphole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine, 3-(2-hydroxyéthyl)benzothiazol-3-ium, et 1-(2-hydroxyéthyl)-pyridinium.

Selon l'invention, pour la formule (I), on entend par aryle, un radical aryle pouvant être substitué par un ou plusieurs radicaux alkyle, alcoxy, acyle, cyano, carboxamido, -CO₂H, -SO₃H, -PO₃H₂, -PO₄H₂, hydroxyle, amino, monoalkyl(C₁-C₄)amino, ou dialkyl(C₁-C₄)amino dans lequel les deux groupements alkyle peuvent former, conjointement avec l'atome d'azote dudit groupement dialkyl(C₁-C₄)amino auquel ils sont liés, un cycle pouvant être interrompu par un ou plusieurs atomes d'azote, d'oxygène ou de soufre. De préférence, le groupe aryle est un groupe phényle pouvant être substitué comme indiqué ci dessus.

Parmi les groupements de formule (II) ci-dessus, on peut notamment citer les groupement acétamide, diméthylurée, O-méthylcarbamate, méthylcarbonate, N-diméthylcarbamate et les esters.

La composition selon l'invention comprend de préférence les 3-amino pyrazolo-[1,5-a]-pyridines répondant à la formule (la) suivante, ainsi que leurs sels d'addition avec un acide ou avec une base : 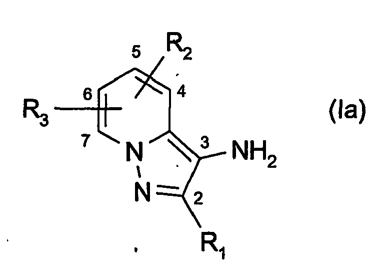 dans laquelle :
R₁, R₂, R₃, identiques ou différents, représentent un atome d'hydrogène ou d'halogène ; un radical -NHSO₃H ; un radical hydroxyle ; un radical alkyle ; un radical alkoxy ; un radical alkylthio ; un radical amino ; un radical monoalkylamino ; un radical dialkylamino dans lequel les deux groupements alkyle peuvent former conjointement avec l'atome d'azote auquel ils sont liés, un cycle pouvant être interrompu par un ou plusieurs atomes d'azote, d'oxygène ou de soufre ; un hétérocycle ; un radical acyle ; un radical alkoxycarbonyle ; un radical carboxamido ; ou un groupement de formule (II) suivante 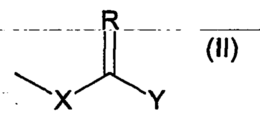
dans laquelle X et R représentent, indépendamment l'un de l'autre, un atome d'oxygène, un groupement NH ou N(C₁-C₄)alkyle, et Y représente un radical hydroxyle, amino, alkyle, alkoxy, alkylamino, ou dialkylamino.

Parmi les 3-amino pyrazolo-[1,5-a]-pyridines de formule (I) utiles à titre de base d'oxydation dans les compositions tinctoriales de l'invention, on peut notamment citer :
- la pyrazolo[1,5-a]pyridin-3-ylamine ;
- la 2-acétylamino pyrazolo-[1,5-a] pyridin-3-ylamine ;
- la 2-morpholin-4-yl-pyrazolo[1,5-a]pyridin-3-ylamine ;
- l'acide 3-amino-pyrazolo[1,5-a]pyridin-2-carboxylique ;
- la 2-méthoxy-pyrazolo[1,5-a]pyridine-3-ylamino ;
- le (3-amino-pyrazolo[1,5-a]pyridine-7-yl)-méthanol ;
- le 2-(3-amino-pyrazolo[1,5-a]pyridine-5-yl)-éthanol ;
- le 2-(3-amino-pyrazolo[1,5-a]pyridine-7-yl)-éthanol ;
- le (3-amino-pyrazolo[1,5-a]pyridine-2-yl)-méthanol ;
- la 3,6-diamino-pyrazolo[1,5-a]pyridine ;
- la 3,4-diamino-pyrazolo[1,5-a]pyridine ;
- la pyrazolo[1,5-a]pyridine-3,7-diamine ;
- la 7-morpholin-4-yl-pyrazolo[1,5-a]pyridin-3-ylamine;
- la pyrazolo[1,5-a]pyridine-3,5-diamine ;
- la 5-morpholin-4-yl-pyrazolo[1,5-a]pyridin-3-ylamine ;
- le 2-[(3-amino-pyrazolo[1,5-a]pyridin-5-yl)-(2-hydroxyéthyl)-amino]-éthanol ;
- le 2-[(3-amino-pyrazolo[1,5-a]pyridin-7-yl)-(2-hydroxyéthyl)-amino]-éthanol ;
- la 3-amino-pyrazolo[1,5-a]pyridine-5-ol ;
- la 3-amino-pyrazolo[1,5-a]pyridine-4.-ol ;
- la 3-amino-pyrazolo[1,5-a]pyridine-6-ol ;
- la 3-amino-pyrazolo[1,5-a]pyridine-7-ol ;
ainsi que leurs sels d'addition avec un acide ou avec une base.

Les 3-amino pyrazolo-[1,5-a]-pyridines de formule (I) sont des composés connus, en particulier dans le domaine pharmaceutique, et sont décrites notamment dans le brevet US 5,457,200. Ces composés peuvent être préparés selon des méthodes de synthèse bien connues dans la littérature et telles que décrites par exemple dans le brevet US 5,457,200.

La ou les 3-amino pyrazolo-[1,5-a]-pyridines de formule (I) ci-dessus et/ou le ou leurs sels d'addition avec un acide ou une base représentent de préférence de 0,0001 à 10 % en poids environ du poids total de la composition tinctoriale, et encore plus préférentiellement de 0,005 à 6 % en poids environ de ce poids.

Selon l'invention, le coupleur pyridinique peut être choisi parmi la 2-amino-3-hydroxypyridine ou la 2-amino-3-hydroxy 5-chloro-pyridine.

Les coupleurs pyridiniques de la présente invention sont connus de la technique et peuvent être préparés selon des synthèses classiques.

Le coupleur pyridinique est de préférence présent dans la composition en quantité comprise entre 0,0001 à 10 % en poids environ du poids total de la composition tinctoriale et encore plus préférentiellement de 0,005 à 6 % en poids environ de ce poids.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C1-C4, tels que l'éthanol et l'isopropanol, les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 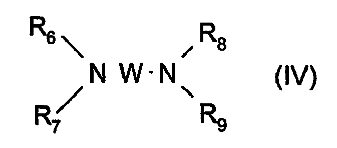 dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

La composition selon l'invention peut contenir en plus du coupleur pyridinique de formule (III), des coupleurs additionnels conventionnellement utilisés pour la teinture de fibres kératiniques. Parmi ces coupleurs, on peut notamment citer les métaphénylènediamines, les méta-aminophénols, les métadiphénols, les coupleurs naphtaléniques et les coupleurs hétérocycliques.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylénedioxybenzène, l'α-naphtol, le 2 méthyl-1-naphtol, le 6-hydroxy indole, le 4-hydroxy indole, le 4-hydroxy N-méthyl indole, la 6-hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Lorsqu'ils sont présents, le ou les coupleurs additionnels représentent de préférence de 0,0001 à 10 % en poids environ du poids total de la composition tinctoriale et encore plus préférentiellement de 0,005 à 6 % en poids environ de ce poids.

La composition tinctoriale conforme à l'invention peut encore contenir au moins une base d'oxydation additionnelle, et qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques différentes des 3-amino pyrazolo-[1,5-a]-pyridines de formule (I) utilisées conformément à l'invention.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylénediamine, la 4-aminophényl pyrrolidine, la 2-thienyl paraphénylènediamine, le 2-β-hydroxy éthylamino-5-aminotoluène et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènèdiamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl). N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques différents des composés de formule (I) conformes à l'invention, les dérivés pyrimidiniques et les dérivés pyrazoliques.

Parmi les dérivés pyridiniques différents des composés de formule (I) conformes à l'invention, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxymétfiyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, la ou les bases d'oxydation additionnelles représentent de préférence de 0,0001 à 10 % en poids environ du poids total de la composition tinctoriale, et encore plus préférentiellement de 0,005 à 6 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates, les citrates, les succinates, les tartrates, les lactates, les phosphates et les acétates. Les sels d'addition avec une base utilisables dans le cadre des compositions tinctoriales de l'invention sont notamment ceux obtenus avec la soude, la potasse, l'ammoniaque ou les amines.

La composition tinctoriale conforme à l'invention peut en outre renfermer un ou plusieurs colorants directs pouvant notamment être choisis parmi les colorants nitrés de la série benzénique, les colorants directs cationiques, en particulier les colorants azoiques ou méthiniques.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces composés peuvent être présents en quantité comprise entre 0,01 et 20 % en poids du poids total de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas ou substantiellement pas altérées par la ou les adjonctions envisagées.

La composition tinctoriale selon l'invention peut se présenter sous des formes diverses, telles que sous forme de liquides, de crèmes, de gels ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à l'aide d'un agent oxydant. La couleur peut être révélée à pH acide, neutre ou alcalin et l'agent oxydant peut être ajouté juste au moment de l'emploi à la composition tinctoriale ou dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition contenant, dans un milieu approprié pour la teinture, au moins un agent oxydant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides et les enzymes parmi lesquelles on peut citer les peroxydases, les oxydo-réductases à 2 électrons telles que les uricàses et les oxygénases à 4 électrons comme les laccases. Le peroxyde d'hydrogène est particulièrement préféré.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture dans lequel un premier compartiment renferme la composition tinctoriale définie ci-dessus et un deuxième compartiment renferme la composition oxydante. Ce dispositif peut être équipé d'un moyen permettant de délivrer sur les cheveux le mélange souhaité, tel que les dispositifs décrits dans le brevet FR-2 586 913 au nom de la demanderesse.

L'invention a enfin pour objet le produit coloré susceptible d'être obtenu par réaction de la base de formule (I) telle que définie précédemment et le coupleur de formule (III) telle que définie précédemment. Ce produit coloré peut être utilisé dans des produits cosmétiques par exemple de maquillage.

Les exemples qui suivent sont destinés à illustrer l'invention.

### EXEMPLES DE TEINTURE

### Exemple 1. Coloration en milieu neutre pH=7

On a préparé la composition de teinture, conforme à l'invention, suivante :

| | |
|---|---|
| - Pyrazolo[1,5-a]pyridin-3-ylamine monochlorhydratée | 0,848 g |
| - 2-amino-3-hydroxypyridine | 0,55 g |
| - Alcool oléique polyglycérolé à 4 moles de glycérol, à 78 % de matières actives (M.A.) | 5,7 g M.A. |
| - Alcool oléique polyglycérolé à 2 moles de glycérol | 4,0 g |
| - Acide oléique | 3,0 g |
| - Amine oléique à 2 moles d'oxyde d'éthylène, vendue sous la dénomination Ethomeen 012 par la Société AKZO | 7,0 g |
| - Laurylamino succinamate de diéthylaminopropyle, sel de sodium, à 55 % de M.A. | 3,0 |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35 % de M.A. | 0,46 g M.A. |
| - Acétate d'ammonium | 0,8 g |
| - Antioxydant, séquestrant, | q.s. |
| - Parfum, conservateur, | q.s. |
| - Acide citrique q s p | pH 7,6. |
| - Eau déminéralisée q.s.p. | 100,0 g |

Au moment de l'emploi, on mélange cette composition poids pour poids avec de l'eau oxygénée titrant 20 volumes (6% en poids), de pH 3. Le pH du mélange est de 7. On applique ce mélange sur des cheveux gris naturels à 90 % de blancs. Après 30 min. de pose, les mèches sont rincées, lavées au shampooing, rincées à nouveau et séchées. Les cheveux sont teints avec un reflet rouge cuivré

## Revendications

1. Composition pour la teinture d'oxydation des fibres kératiniques, **caractérisée en ce qu'**elle comprend, dans un milieu approprié pour la teinture,
• à titre de base d'oxydation au moins une 3-amino pyrazolo-[1,5-a]-pyridine de formule (I) suivante et/ou un de ses sels d'addition avec un acide ou avec une base: 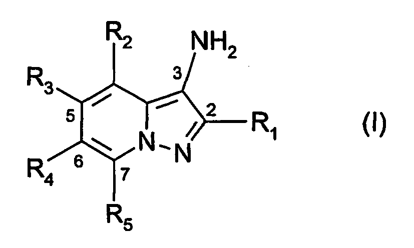 dans laquelle :
- R₁, R₂, R₃, R₄ et R₅, identiques ou différents, représentent un atome d'hydrogène ou d'halogène ; un radical -NHSO₃H ; un radical hydroxyle ; un radical alkyle ; un radical alkoxy ; un radical alkylthio ; un radical amino ; un radical monoalkylamino ; un radical dialkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle ; un radical nitro ; un radical aryle ; un radical acyle en C1-C4 ; un radical alkoxy(C₁-C₄)carbonyle ; un radical carboxamido ; un radical cyano ; un radical -CO₂H, un radical -SO₃H ; un radical -PO₃H₂ ; un radical -PO₄H₂; ou un groupement de formule (II) suivante : 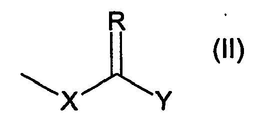
dans laquelle X et R représentent indépendamment l'un de l'autre un atome d'oxygène, un groupement NH ou N(C₁-C₄)alkyle, et Y représente un radical hydroxyle, amino, alkyle, alkoxy, alkylamino, ou dialkylamino, et
• au moins un coupleur pyridinique de formule (III) suivante : 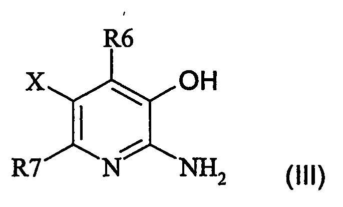 dans laquelle
- R6 et R7, identiques ou différents, représentent un atome d'hydrogène ; un radical alkyle en C1-C4, et
- X est un atome d'hydrogène ou d'halogène.

2. Composition selon la revendication 1, **caractérisée en ce que** les composés de formule (I) sont choisis parmi les composés de formule (la) suivante, et leurs sels d'addition avec un acide ou avec une base : 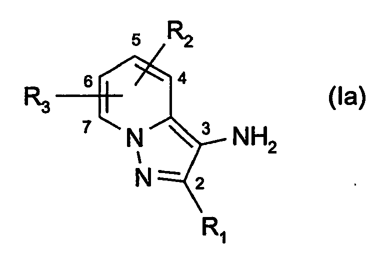 dans laquelle :
R₁,R₂, R₃, identiques ou différents, représentent un atome d'hydrogène.ou d'halogène ; un radical -NHSO₃H ; un radical hydroxyle ; un radical alkyle ; un radical alkoxy ; un radical alkylthio ; un radical amino, un radical monoalkylamino ; un radical dialkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle ; un radical acyle ; un radical alkoxycarbonyle ; un radical carboxamido ; ou un groupement de formule (II) suivante 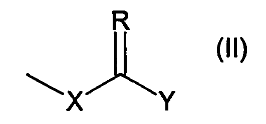
dans laquelle X et R représentent indépendamment l'un de l'autre un atome d'oxygène, un groupement NH ou N(C₁-C₄)alkyle, et Y représente un radical hydroxyle, amino, alkyle, alkoxy, alkylamino, ou dialkylamino.

3. Composition selon la revendications 1 ou 2 dans laquelle les 3-amino pyrazolo-[1,5-a]-pyridines de formule (I) sont choisies parmi :
- la pyrazolo[1,5-a]pyridin-3-ylamine ;
- la 2-acétylamino pyrazolo-[1,5-a] pyridin-3-ylamine ;
- la 2-morpholin-4-yl-pyrazolo[1,5-a]pyridin-3-ylamine;
- l'acide 3-amino-pyrazolo[1,5-a]pyridin-2-carboxylique ;
- la 2-méthoxy-pyrazolo[1,5-a]pyridine-3-ylamino ;
- le (3-amino-pyrazolo[1,5-a]pyridine-7-yl)-méthanol ;
- le 2-(3-amino-pyrazolo[1,5-a]pyridine-5-yl)-éthanol;
- le 2-(3-amino-pyrazolo[1,5-a]pyridine-7-yl)-éthanol;
- le (3-amino-pyrazolo[1,5-a]pyridine-2-yl)-méthanol ;
- la 3,6-diamino-pyrazolo[1,5-a]pyridine;
- la 3,4-diamino-pyrazolo[1,5-a]pyridine ;
- la pyrazolo[1,5-a]pyridine-3,7-diamine ;
- la 7-morpholin-4-yl-pyrazolo[1,5-a]pyridin-3-ylamine ;
- la pyrazolo[1,5-a]pyridine-3,5-diamine ;
- la 5-morpholin-4-yl-pyrazolo[1,5-a]pyridin-3-ylamine ;
- le 2-[(3-amino-pyrazolo[1,5-a]pyridin-5-yl)-(2-hydroxyéthyl)-amino]-éthanol ;
- le 2-[(3-amino-pyrazolo[1,5-a]pyridin-7-yl)-(2-hydroxyéthyl)amino]-éthanol ;
- la 3-amino-pyrazolo[1,5-a]pyridine-5-ol;
- la 3-amino-pyrazolo[1,5-a]pyridine-4-ol ;
- la 3-amino-pyrazolo[1,5-a]pyridine-6-ol ;
- la 3-amino-pyrazolo[1,5-a]pyridine-7-ol ;
et leurs d'addition avec un acide ou avec une base.

4. Composition selon l'une quelconque des revendications précédentes, **caractérisée en ce que** la ou les 3-amino pyrazolo-[1,5-a]-pyridines de formule (I) et/ou le ou leurs sels d'addition avec un acide ou une base représentent de 0,0001 à 10 % en poids du poids total de la composition tinctoriale.

5. Composition selon la revendication 4, **caractérisée en ce que** la ou les 3-amino pyrazolo-[1,5-a]-pyridines de formule (I) et/ou le ou leurs sels d'addition avec un acide ou une base représentent de 0,005 à 6 % en poids du poids total de la composition tinctoriale.

6. Composition selon l'une quelconque des revendications 1 à 5 dans laquelle le coupleur de formule (III) est la 2-amino 3-hydroxypyridine ou la 2-amino 3-hydroxy 5-chloro pyridine.

7. Composition selon l'une quelconque des revendications précédentes **caractérisée en ce que** le coupleur de formule (III) est présent en quantité comprise entre 0,0001 à 10 % en poids environ du poids total de la composition tinctoriale.

8. Composition selon l'une quelconque des revendications précédentes, comprenant de plus au moins une base d'oxydation additionnelle choisie parmi les paraphénylènediamines, les bis-phénylalkylènediamines, les para-aminophénols, les ortho-aminophénols et les bases hétérocycliques différentes des 3-amino pyrazolo-[1,5-a]-pyridines de formule (I) telle que définie à la revendication 1.

9. Composition selon l'une quelconque des revendications précédentes, comprenant de plus un ou plusieurs coupleurs additionnels choisis parmi les métaphénylènediamines, les méta-aminophénols, les métadiphénols, les coupleurs naphtaléniques et les coupleurs hétérocycliques.

10. Composition selon la revendication 9, **caractérisée en ce que** les coupleurs additionnels sont choisis parmi le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylénedioxybenzène, l'α-naphtol, le 2 méthyl-1-naphtol, le 6-hydroxy indole, le 4-hydroxy indole, le 4-hydroxy N-méthyl indole, la 6-hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

11. Procédé de teinture d'oxydation des fibres kératiniques, **caractérisé en ce qu'**on applique sur les fibres au moins une composition tinctoriale telle que définie à l'une quelconque des revendications 1 à 10, et que l'on révèle la couleur en présence d'un agent oxydant.

12. Procédé selon la revendication 11, **caractérisé en ce que** l'agent oxydant est choisi parmi le peroxyde d'hydrogène, le peroxyde d'urée, les bromates de métaux alcalins, les persels, les peracides, et les enzymes.

13. Dispositif à plusieurs compartiments comprenant un premier compartiment qui renferme une composition tinctoriale telle que définie à l'une quelconque des revendications 1 à 10 et un deuxième compartiment qui renferme une composition contenant un agent oxydant.

14. Produit coloré susceptible d'être obtenu par réaction d'une base d'oxydation de formule (I) et du coupleur de formule (III) à partir de la composition selon l'une quelconque des revendications 1 à 10.

## Claims

1. Composition for the oxidation dyeing of keratinous fibres, **characterized in that** it comprises, in a medium appropriate for dyeing,
• as oxidation base, at least one 3-aminopyrazolo[1,5-a]pyridine of following formula (I) and/or one of its addition salts with an acid or with a base: 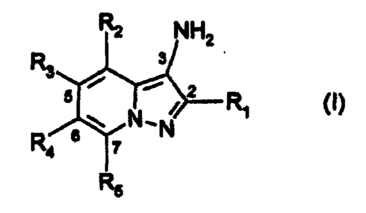 in which:
- R₁, R₂, R₃, R₄ and R₅, which are identical or different, represent a hydrogen or halogen atom; an -NHSO₃H radical; a hydroxyl radical; an alkyl radical; an alkoxy radical; an alkylthio radical; an amino radical; a monoalkylamino radical; a dialkylamino radical in which the two alkyl groups can, together with the nitrogen atom to which they are bonded, form a ring which can be interrupted by one or more nitrogen, oxygen or sulphur atoms; a heterocycle; a nitro radical; an aryl radical; a C₁-C₄ acyl radical; a (C₁-C₄) alkoxycarbonyl radical; a carboxamido radical; a cyano radical; a -CO₂H radical; an -SO₃H radical; a -PO₃H₂ radical; a
- PO₄H₂ radical; or a group of following formula (II) : 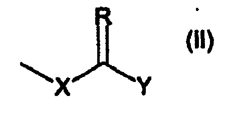 in which X and R represent, independently of one another, an oxygen atom or an NH or N (C₁-C₄) alkyl group, and Y represents a hydroxyl, amino, alkyl, alkoxy, alkylamino or dialkylamino radical, and
• at least one pyridine coupler of following formula (III): 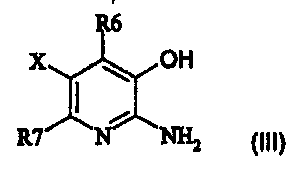 in which:
- R6 and R7, which are identical or different, represent a hydrogen atom or a C₁-C₄ alkyl radical, and
- X is a hydrogen or halogen atom.

2. Composition according to Claim 1, **characterized in that** the compounds of formula (I) are chosen from the compounds of following formula (Ia) and their addition salts with an acid or with a base: 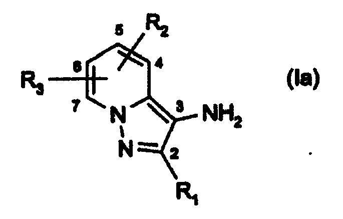 in which:
R₁, R₂ and R₃, which are identical or different, represent a hydrogen or halogen atom; an -NHSO₃H radical; a hydroxyl radical; an alkyl radical; an alkoxy radical; an alkylthio radical; an amino radical; a monoalkylamino radical; a dialkylamino radical in which the two alkyl groups can, together with the nitrogen atom to which they are bonded, form a ring which can be interrupted by one or more nitrogen, oxygen or sulphur atoms; a heterocycle; an acyl radical; an alkoxycarbonyl radical; a carboxamido radical; or a group of following formula (II): 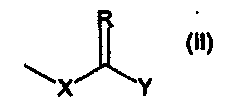
in which X and R represent, independently of one another, an oxygen atom or an NH or N(C₁-C₄) alkyl group, and Y represents a hydroxyl, amino, alkyl, alkoxy, alkylamino or dialkylamino radical.

3. Composition according to Claim 1 or 2, in which the 3-aminopyrazolo[1,5-a]pyridines of formula (I) are chosen from:
- pyrazolo[1,5-a]pyridin-3-ylamine;
- 2-(acetylamino)pyrazolo[1,5-a]pyridin-3-ylamine;
- 2-(morpholin-4-yl)pyrazolo[1,5-a]pyridin-3-ylamine;
- 3-aminopyrazolo[1,5-a]pyridine-2-carboxylic acid;
- 2-methoxypyrazolo[1,5-a]pyridin-3-ylamine;
- (3-aminopyrazolo[1,5-a]pyridin-7-yl)methanol;
- 2-(3-aminopyrazolo[1,5-a]pyridin-5-yl)ethanol;
- 2-(3-aminopyrazolo[1,5-a]pyridin-7-yl)ethanol;
- (3-aminopyrazolo[1,5-a]pyridin-2-yl)methanol;
- 3,6-diaminopyrazolo[1,5-a]pyridine;
- 3,4-diaminopyrazolo[1,5-a]pyridine;
- pyrazolo[1,5-a]pyridine-3,7-diamine;
- 7-(morpholin-4-yl)pyrazolo[1,5-a]pyridin-3-ylamine;
- pyrazolo[1,5-a]pyridine-3,5-diamine;
- 5-(morpholin-4-yl)pyrazolo[1,5-a]pyridin-3-ylamine;
- 2-[(3-aminopyrazolo[1,5-a]pyridin-5-yl)(2-hydroxyethyl)amino]ethanol;
- 2-[(3-aminopyrazolo[1,5-a]pyridin-7-yl)(2-hydroxyethyl)amino]ethanol;
- 3-aminopyrazolo[1,5-a]pyridin-5-ol;
- 3-aminopyrazolo[1,5-a]pyridin-4-ol;
- 3-aminopyrazolo[1,5-a]pyridin-6-ol;
- 3-aminopyrazolo[1,5-a]pyridin-7-ol;
and their addition salts with an acid or with a base.

4. Composition according to any one of the preceding claims, **characterized in that** the 3-amino-pyrazolo[1,5-a]pyridine compound or compounds of formula (I) and/or the addition salt or their addition salts with an acid or a base represent from 0.0001 to 10% by weight of the total weight of the dyeing composition.

5. Composition according to Claim 4, **characterized in that** the 3-aminopyrazolo[1,5-a]pyridine compound or compounds of formula (I) and/or the addition salt or their addition salts with an acid or a base represent from 0.005 to 6% by weight of the total weight of the dyeing composition.

6. Composition according to any one of Claims 1 to 5, in which the coupler of formula (III) is 2-amino-3-hydroxypyridine or 2-amino-3-hydroxy-5-chloro-pyridine.

7. Composition according to any one of the preceding claims, **characterized in that** the coupler of formula (III) is present in an amount of between 0.0001 and 10% by weight approximately of the total weight of the dyeing composition.

8. Composition according to any one of the preceding claims, additionally comprising at least one additional oxidation base chosen from para-phenylenediamines, bisphenylalkylenediamines, para-aminophenols, ortho-aminophenols and heterocyclic bases other than the 3-amino-pyrazolo[1,5-a]pyridines of formula (I) as defined in Claim 1.

9. Composition according to any one of the preceding claims, additionally comprising one or more additional couplers chosen from meta-phenylenediamines, meta-aminophenols, meta-diphenols, naphthalene couplers and heterocyclic couplers.

10. Composition according to Claim 9, **characterized in that** the additional couplers are chosen from 2-methyl-5-aminophenol, 5-[N-(β-hydroxyethyl)-amino]-2-methylphenol, 6-chloro-2-methyl-5-aminophenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)-benzene, 2-amino-4-(β-hydroxyethylamino)-1-methoxybenzene, 1,3-diaminobenzene, 1,3-bis-(2,4-diaminophenoxy)propane, 3-ureidoaniline, 3-ureido-1-(dimethylamino)benzene, sesamol, 1-(β-hydroxyethylamino)-3,4-(methylenedioxy)-benzene, α-naphthol, 2-methyl-1-naphthol, 6-hydroxyindole, 4-hydroxyindole, 4-hydroxy-N-methylindole, 6-hydroxybenzomorpholine, 3,5-diamino-2,6-dimethoxypyridine, 1-[N-(β-hydroxyethyl)amino]-3,4-(methylenedioxy)-benzene, 2,6-bis(β-hydroxyethylamino)toluene and their addition salts.

11. Method for the oxidation dyeing of keratinous fibres, **characterized in that** at least one dyeing composition as defined in any one of Claims 1 to 10 is applied to the fibres and **in that** the colour is developed in the presence of an oxidizing agent.

12. Method according to Claim 11, **characterized in that** the oxidizing agent is chosen from hydrogen peroxide, urea hydrogen peroxide, alkali metal bromates, persalts, peracids and enzymes.

13. Multicompartment device comprising a first compartment which includes a dyeing composition as defined in any one of Claims 1 to 10 and a second compartment which includes a composition comprising an oxidizing agent.

14. Coloured product capable of being obtained by reaction of an oxidation base of formula (I) and the coupler of formula (III) starting from the composition according to any one of Claims 1 to 10.

## Patentansprüche

1. Zusammensetzung zum oxidativen Färben von Keratinfasern, **dadurch gekennzeichnet, dass** sie in einem zum Färben geeigneten Medium enthält:
• als Oxidationsbase mindestens ein 3-Amino-pyrazolo-[1,5-a]-pyridin der folgenden Formel (I) und/oder eines seiner Additionssalze mit einer Säure oder einer Base der Formel: 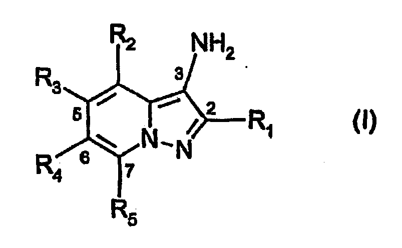 in der Formel:
- R₁, R₂, R₃, R₄ und R₅, die gleich oder verschieden sind, bedeuten ein Wasserstoffatom oder ein Halogenatom; eine Gruppe -NHSO₃H; eine Hydroxygruppe; eine Alkylgruppe; eine Alkoxygruppe; eine Alkylthiogruppe; eine Aminogruppe; eine Monoalkylaminogruppe; eine Dialkylaminogruppe, bei der die beiden Alkylgruppen gemeinsam mit dem Stickstoffatom, an das sie gebunden sind, einen Ring bilden können, der durch ein oder mehrere Stickstoffatome, Sauerstoffatome oder Schwefelatome unterbrochen sein kann; einen Heterocyclus; eine Nitrogruppe; eine Arylgruppe; eine C₁₋₄-Acylgruppe; eine Alkoxy(C₁₋₄)carbonylgruppe; eine Carboxamidogruppe; eine Cyanogruppe; eine Gruppe -CO₂H, eine Gruppe -SO₃H; eine Gruppe PO₃H₂; eine Gruppe -PO₄H₂; oder eine Gruppe der folgenden Formel (II) : 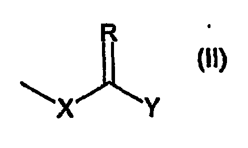 worin X und R unabhängig voneinander ein Sauerstoffatom, eine Gruppe NH oder N(C₁₋₄)Alkyl bedeuten und Y eine Gruppe Hydroxy, Amino, Alkyl, Alkoxy, Alkylamino oder Dialkylamino ist, und
• mindestens einen Pyridinkuppler der folgenden Formel (III): 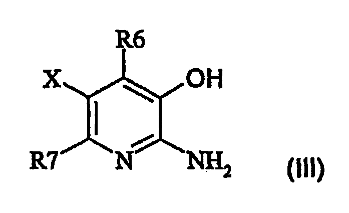 in der Formel:
- R6 und R7, die gleich oder verschieden sind, bedeuten ein Wasserstoffatom; eine C₁₋₄-Alkylgruppe, und
- X ist ein Wasserstoffatom oder Halogenatom.

2. Zusammensetzung nach Anspruch 1, **dadurch gekennzeichnet, dass** die Verbindungen der Formel (I) unter den Verbindungen der folgenden Formel (Ia) und deren Additionssalzen mit einer Säure oder mit einer Base ausgewählt sind: 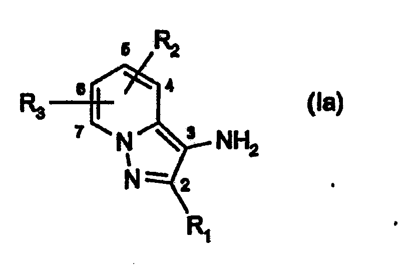 in der Formel:
- die Gruppen R₁, R₂ und R₃, die gleich oder verschieden sind, bedeuten ein Wasserstoffatom oder ein Halogenatom; eine Gruppe -NHSO₃H; eine Hydroxygruppe; eine Alkylgruppe; eine Alkoxygruppe; eine Alkylthiogruppe; eine Aminogruppe, eine Monoalkylaminogruppe; eine Dialkylaminogruppe, bei der die beiden Alkylgruppen gemeinsam mit dem Stickstoffatom, an das sie gebunden sind, einen Ring bilden können, der durch ein oder mehrere Stickstoffatome, Sauerstoffatome oder Schwefelatome unterbrochen sein kann; einen Heterocyclus, eine Acylgruppe; eine Alkoxycarbonylgruppe; eine Carboxamidogruppe; oder eine Gruppe der folgenden Formel (II): 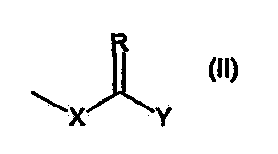 worin X und R unabhängig voneinander ein Sauerstoffatom, eine Gruppe NH oder N(C₁₋₄)Alkyl bedeuten und Y eine Gruppe Hydroxy, Amino, Alkyl, Alkoxy, Alkylamino oder Dialkylamino ist.

3. Zusammensetzung nach Anspruch 1 oder 2, bei der die 3-Amino-pyrazolo-[1,5-a]-pyridine der Formel (I) ausgewählt sind unter:
- Pyrazolo[1,5-a]pyridin-3-ylamin;
- 2-Acetylamino-pyrazolo[1,5-a]pyridin-3-ylamin;
- 2-Morpholin-4-yl-pyrazolo[1,5-a]pyridin-3-ylamin;
- 3-Amino-pyrazolo[1,5-a]pyridin-2-carbonsäure;
- 2-Methoxy-pyrazolo[1,5-a]pyridin-3-ylamin;
- (3-Amino-pyrazolo[1,5-a]pyridin-7-yl)-methanol;
- 2-(3-Amino-pyrazolo[1,5-a]pyridin-5-yl)-ethanol;
- 2-(3-Amino-pyrazolo[1,5-a]pyridin-7-yl)-ethanol;
- (3-Amino-pyrazolo[1,5-a]pyridin-2-yl)-methanol;
- 3,6-Diamino-pyrazolo[1,5-a]pyridin;
- 3,4-Diamino-pyrazolo[1,5-a]pyridin;
- Pyrazolo[1,5-a] pyridin-3,7-diamin;
- 7-Morpholin-4-yl-pyrazolo[1,5-a]pyridin-3-ylamin;
- Pyrazolo[1,5-a]pyridin-3,5-diamin;
- 5-Morpholin-4-yl-pyrazolo[1,5-a]pyridin-3-ylamin;
- 2-[(3-Amino-pyrazolo[1,5-a]pyridin-5-yl)-(2-hydroxyethyl)-amino]-ethanol;
- 2-[(3-Amino-pyrazolo[1,5-a]pyridin-7-yl)-(2-hydroxyethyl)-amino]-ethanol;
- 3-Amino-pyrazolo[1,5-a]pyridin-5-ol;
- 3-Amino-pyrazolo[1,5-a]pyridin-4-ol;
- 3-Amino-pyrazolo[1,5-a]pyridin-6-ol;
- 3-Amino-pyrazolo[1,5-a]pyridin-7-ol;
und deren Additionssalzen mit einer Säure oder mit einer Base.

4. Zusammensetzung nach einem der vorhergehenden Ansprüche, **dadurch gekennzeichnet, dass** das oder die 3-Amino-pyrazolo-[1,5-a]-pyridine der Formel (I) und/oder das oder die Additionssalze dieser Verbindung(en) mit einer Säure oder einer Base 0,0001 bis 10 Gew.-% des Gesamtgewichts der Farbmittelzusammensetzung ausmachen.

5. Zusammensetzung nach Anspruch 4, **dadurch gekennzeichnet, dass** das oder die 3-Amino-pyrazolo-[1,5-a]-pyridine der Formel (I) und/oder das oder die Additionssalze dieser Verbindung(en) mit einer Säure oder einer Base 0,005 bis 6 Gew.-% des Gesamtgewichts der Farbmittelzusammensetzung ausmachen.

6. Zusammensetzung nach einem der Ansprüche 1 bis 5, bei der der Kuppler der Formel (III) das 2-Amino-3-hydroxypyridin oder das 2-Amino-3-hydroxy-5-chlorpyridin ist.

7. Zusammensetzung nach einem der vorhergehenden Ansprüche, **dadurch gekennzeichnet, dass** der Kuppler der Formel (III) in einem Mengenanteil von etwa 0,0001 bis 10 Gew.-% des Gesamtgewichts der Farbmittelzusammensetzung vorliegt.

8. Zusammensetzung nach einem der vorhergehenden Ansprüche, die ferner mindestens eine ergänzende Oxidationsbase enthält, die unter den para-Phenylendiaminen, Bisphenylalkylendiaminen, para-Aminophenolen, ortho-Aminophenolen und den heterocyclischen Basen, die von den 3-Amino-pyrazolo-[1,5-a]-pyridinen der Formel (I) nach Anspruch 1 verschieden sind, ausgewählt sind.

9. Zusammensetzung nach einem der vorhergehenden Ansprüche, die ferner einen oder mehrere ergänzende Kuppler enthält, die unter den meta-Phenylendiaminen, meta-Aminophenolen, meta-Dihydroxybenzolen, Naphthalinkupplern und heterocyclischen Kupplern ausgewählt sind.

10. Zusammensetzung nach Anspruch 9, **dadurch gekennzeichnet, dass** die ergänzenden Kuppler ausgewählt sind unter 2-Methyl-5-aminophenol, 5-N-(β-Hydroxyethyl)amino-2-methylphenol, 6-Chlor-2-methyl-5-aminophenol, 3-Aminophenol, 1,3-Dihydroxybenzol, 1,3-Dihydroxy-2-methylbenzol, 4-Chlor-1,3-dihydroxybenzol, 2,4-Diamino-1-(β-hydroxyethyloxy)-benzol, 2-Amino-4-(β-hydroxyethylamino)-1-methoxybenzol, 1,3-Diaminobenzol, 1,3-Bis(2,4-diaminophenoxy)propan, 3-Ureidoanilin, 3-Ureido-1-dimethylaminobenzol, Sesamol, 1-β-Hydroxyethylamino-3,4-methylendioxybenzol, α-Naphthol, 2-Methyl-1-naphthol, 6-Hydroxyindol, 4-Hydroxyindol, 4-Hydroxy-N-methylindol, 6-Hydroxybenzomorpholin, 3,5-Diamino-2,6-dimethoxypyridin, 1-N-(β-Hydroxyethyl)amino-3,4-methylendioxybenzol, 2,6-Bis(β-hydroxyethylamino)toluol und ihren Additionssalzen.

11. Verfahren zum oxidativen Färben von Keratinfasern, **dadurch gekennzeichnet, dass** auf die Fasern mindestens eine Farbmittelzusammensetzung nach einem der Ansprüche 1 bis 10 aufgebracht und die Farbe in Gegenwart eines Oxidationsmittels gebildet wird.

12. Verfahren nach Anspruch 11, **dadurch gekennzeichnet, dass** das Oxidationsmittel unter Wasserstoffperoxid, Harnstoffperoxid, Alkalimetallbromaten, Salzen von Persäuren, Persäuren und Enzymen ausgewählt ist.

13. Vorrichtung mit mehreren Abteilungen, die eine erste Abteilung umfasst, die eine Farbmittelzusammensetzung nach einem der Ansprüche 1 bis 10 enthält, und eine zweite Abteilung, die eine Zusammensetzung mit einem Oxidationsmittel enthält.

14. Farbiges Produkt, das durch Reaktion einer Oxidationsbase der Formel (I) und eines Kupplers der Formel (III) ausgehend von der Zusammensetzung nach einem der Ansprüche 1 bis 10 erhältlich ist.
